# EUROPEAN PATENT SPECIFICATION

(11) **EP 4 153 059 B1**
(45) Date of publication and mention of the grant of the patent: **29.01.2025**
(21) Application number: 21737325.7
(22) Date of filing: 23.06.2021
(51) Int. Cl.: A61B 6/03, A61B 6/00

(54) **COOLING SYSTEM INTEGRATED WITHIN MODULAR, DETECTOR ELECTRONIC ASSEMBLY FOR A DIAGNOSTIC MEDICAL IMAGING APPARATUS**
IN EINE MODULARE ELEKTRONISCHE DETEKTORANORDNUNG INTEGRIERTES KÜHLSYSTEM FÜR EINE MEDIZINISCHE DIAGNOSTISCHE BILDGEBUNGSVORRICHTUNG
SYSTÈME DE REFROIDISSEMENT INTÉGRÉ À L'INTÉRIEUR D'UN ENSEMBLE ÉLECTRONIQUE DE DÉTECTEUR MODULAIRE POUR UN APPAREIL D'IMAGERIE MÉDICALE DE DIAGNOSTIC

(30) Priority: 25.06.2020 US 202016946514; 26.08.2020 WO PCT/US2020/070462; 28.09.2020 US 202063198079 P
(43) Date of publication of application: 29.03.2023
(73) Proprietor: Siemens Medical Solutions USA, Inc., Malvern, PA 19355 (US)
(72) Inventor: BURBAR, Ziad, Knoxville, Tennessee 37932 (US); KELLER, John, Knoxville, Tennessee 37923 (US); MOOR, Andrew Philip, Knoxville, Tennessee 37932 (US); CORBEIL, James L., Knoxville, Tennessee 37922 (US)
(74) Representative: Horn Kleimann Waitzhofer Schmid-Dreyer Patent- und Rechtsanwälte PartG mbB
(86) International application number: PCT/US2021/070752
(87) International publication number: WO 2021/263275

(56) References cited:
- CN-U- 210 130 852
- US-A1- 2012 091 341
- US-A1- 2013 284 936

## Description

This application is a continuation in part of and claims the benefit of priority of an International Application filed in the United States Receiving Office, entitled "Cooling Channel With Non-Metallic Heat Sink For A Diagnostic Medical Imaging Apparatus", filed August 26, 2020 and assigned serial number PCT/US2020/070462. This application is also a continuation in part of and claims the benefit of priority to a United States Application entitled "Method And Apparatus For Mounting And Aligning Detectors Of A Medical Imaging Apparatus", filed June 25, 2020 and assigned serial number 16/946,514.

### TECHNICAL FIELD

This disclosure relates to cooling systems for medical imaging apparatuses. More particularly, this disclosure relates to modular, scalable fluid-cooling systems and methods for cooling gantries of medical imaging apparatuses, including gantries of long, axial field of view apparatuses.

### BACKGROUND

Diagnostic medical imaging apparatuses include, by way of non-limiting example, computed tomography (CT), two-dimensional digital radiography (DR), magnetic resonance imaging (MRI), positron emission tomography (PET), single photon emission computed tomography (SPECT) modalities. Hybrid modality apparatuses include, by way of non-limiting example, PET/CT, PET/MRI, SPECT/CT, and SPECT/MRI, which combine in a single system the local imaging resolution benefits of CT or MRI and the sensitivity for imaging and detecting cellular and metabolic biological processes in a patient. Many of these imaging apparatuses or systems include a toroidal-shaped gantry structure through which is inserted a patient table. The gantry includes one or more circumferential rows and axially oriented columns of electromagnetic radiation detectors, which form a matrix-like detector array. The respective radiation detectors in the detector array emit electrons in response to incident photons of electromagnetic radiation. In some modalities, the incident photons are transmitted X-rays or ionized radiation emissions at the higher end of the electromagnetic frequency range (e.g., CT, DR, PET, SPECT), while in other modalities (e.g., MRI) the incident photons are within the radio frequency range. The output electrons of the detector elements in the detectors are processed by detector electronics to generate detector output signals, which are subsequently processed by the imaging apparatus to generate or construct patient images. In some imaging systems, detector electronics packages are housed with the detectors within the gantry structure in an integrated detector assembly.

Exemplary electromagnetic radiation detectors include photomultiplier tubes (PMTs) and solid-state detectors, such as avalanche photo diodes (APDs) and silicon photomultipliers (SiPMs). Signal gain of solid-state detectors are more temperature dependent than PMTs. The solid-state photon sensors and their detector electronics packages are typically maintained within relatively narrow temperature fluctuation and operational temperature bandwidths to reduce the likelihood of inaccurate detector readings and/or excessive noise generation components in the detector readings that otherwise might lead to poor quality patient images. The solid-state radiation detectors require external cooling to maintain detector assemblies within defined temperature fluctuation and bandwidth specifications. Typically, radiation detectors and detector assemblies in medical imaging systems are cooled by blowing cooling air over them, or by transferring detector heat to one or more conduits routed about the gantry structure that circulate cooling fluid in proximity to them.

In past cooling system designs for exemplary PET/CT scanning modalities, wherein the imaging apparatus gantry incorporates a PET axial field of view (aFOV) scanning zone that is axially aligned with a CT aFOV scanning zone, the cooling system was specifically sized to transfer heat from only one or two circumferential rows and axial columns of detector assemblies. Blown cooling air systems were satisfactory for such applications. However, recent movement in the field of PET imaging, with aFOV expansion of the patient imaging approaching one to two meters, has made it necessary to revisit the nature of both the design and the fabrication of such cooling systems, including in exemplary PET/CT systems. Often these axially expanded systems require cooling of a plurality of greater than two rows in each column of detector assemblies, further complicating blown cooling air-type cooling system architecture. Past cooling system designs for longer aFOV imaging systems have concatenated two or more existing, shorter aFOV cooling systems within the same gantry. In other words, conventional, known cooling system designs, modified for longer aFOV imaging systems, have utilized multiple water-to-air heat exchangers with fans to blow cooled air within the gantry, to transfer the heat out of the system back to the heat exchanger. This introduces two complexities to the design. The first one is limited space in the system. To remove the heat efficiently, the design might require adding larger and/or more heat exchangers in the gantry of the system. The second is related to noise generated due to the increased number of fans and the air flow required to remove the heat from the gantry of the extended aFOV imaging system. It becomes economically averse to fabricate medical imaging system gantry cooling systems for different combinations and orientations of detector assemblies (e.g., standard FOV systems with one or two /rows of such assemblies in each axially aligned column, versus extended, aFOV systems with more than two rows of detector assemblies per axial column).

Document US2012091341A discloses cooling units for imaging systems.

### SUMMARY

Exemplary cooling system embodiments described herein are modular and scalable to accommodate varying numbers of detector assembly orientations and geometries within gantry architectures of different medical imaging systems. Fluid cooled, modular components of the cooling systems are incorporated within individual detector electronic assemblies (DEAs). Exemplary coolant fluids include compressible and incompressible fluids such as liquids and gases (e.g., room air, nitrogen, water) or phase-change refrigerants. Each DEA includes therein a first chill plate for cooling detector elements and a second chill plate for cooling electronic components, such as printed circuit boards and/or power supplies. In some embodiments, each DEAs' first chill plate is thermally conductively coupled to cooling detector elements therein and the second chill plate is thermally conductively coupled to the other electronic components therein. Coolant flow cascades sequentially through the first chill plate and then through the second chill plate. In some embodiments, plural DEAs are interconnected in cascaded fashion, sharing a common, scalable coolant flow path. In various embodiments, any desired number of rows and columns of DEAs are selectively interconnected within the coolant flow path. In some embodiments, components of the fluid cooling system, such as liquid-liquid heat exchangers, pumps, and flow control valves, are located external the imaging system gantry. External location of such components conserves space within the gantry and reduces likelihood of coolant leak infiltration therein. Beneficially, in some system embodiments, including longer aFOV systems, flexible scaling of higher gantry heat loads is achieved, by increasing or decreasing the heat transfer capability of the external cooling system components in proportion to the number of DEAs within the gantry.

Aspects of this disclosure are directed to a fluid coolant system for a gantry of a medical imaging apparatus where the cooling system cools scalable detector electronic assemblies (DEAs) within the gantry. Each DEA includes within its modular housing a first chill plate thermally conductively coupled to cooling detector elements therein and a separate, second chill plate thermally conductively coupled to other electronic components, such as printed circuit boards and/or power supplies therein. In some embodiments, the first chill plate is oriented between the detector elements and the second chill plate, for thermally isolating the detector elements from other heat dissipating components within the DEA. In some embodiments, one or more of the first or second chill plates are segmented into plural sub segments, sharing a common coolant pipe. In some embodiments, coolant flow cascades sequentially through the first chill plate and then through the second chill plate. In this type of coolant flow path, the detector elements have cooling priority in the DEA over the other cooled components.

Exemplary embodiments disclosed herein feature a fluid coolant system for a medical imaging apparatus, having a gantry forming a patient tunnel; a cooling apparatus, coupled to and external the gantry, having a coolant supply for supplying fluid coolant to the gantry, and a coolant return for returning the coolant to the cooling apparatus. The imaging apparatus includes a detector electronic assembly (DEA) within the gantry, coupled to the cooling apparatus, having a housing; detector elements in the housing, for detecting incident photons of electromagnetic radiation originating outside of the housing and other electronic components in the housing. A fluid cooled, first chill plate is thermally conductively coupled to the detector elements, for cooling the detector elements. The first chill plate has a first inlet for receiving the coolant from the coolant supply and a first outlet for discharging the coolant to the coolant return. The DEA also includes a fluid cooled, second chill plate thermally conductively coupled to the other electronic components, for cooling the other electronic components. The second chill plate has a second inlet for receiving the coolant from the coolant supply and a second outlet for discharging the coolant to the coolant return.

Other exemplary embodiments disclosed herein feature a detector electronic assembly (DEA) for a medical imaging apparatus, including a housing; detector elements retained in the housing, for detecting incident photons of electromagnetic radiation originating outside of the housing; and other electronic components in the housing. The DEA includes a fluid cooled, first chill plate thermally conductively coupled to the detector elements, for cooling the detector elements, with the first chill plate having a first inlet for receiving fluid coolant and a first outlet for discharging the coolant. The DEA also includes a fluid cooled, second chill plate thermally conductively coupled to the other electronic components, for cooling the other electronic components, the second chill plate having a second inlet for receiving the coolant and a second outlet for discharging the coolant. The respective first and second inlets and outlets accessible outside the housing.

Additional exemplary embodiments of disclosed herein feature a method for scalable cooling of a gantry of a medical imaging apparatus, including: providing a gantry forming a patient tunnel; providing a cooling apparatus, coupled to and external the gantry, having a coolant supply for supplying fluid coolant to the gantry, and a coolant return for returning the coolant to the cooling apparatus The method includes providing a selected plurality of detector electronic assemblies (DEAs) for orientation within the gantry, with each respective DEA having a housing; detector elements in the housing, for detecting incident photons of electromagnetic radiation originating outside of the housing; and other electronic components in the housing, including a printed circuit board and a power supply. The provided DEA has a fluid cooled, first chill plate thermally conductively coupled to the detector elements, for cooling the detector elements, which is oriented in the housing intermediate the detector elements and the other electronic components. The first chill plate has a first inlet for receiving the coolant from the coolant supply and a first outlet for discharging the coolant to the coolant return. The provided DEA has a fluid cooled, second chill plate for cooling the other electronic components. The second chill plate has opposed first and second sides, respectively thermally conductively coupled to the printed circuit board on its first side and to the power supply on its second side,. The second chill plate has a second inlet for receiving the coolant from the coolant supply and a second outlet for discharging the coolant to the coolant return. In some embodiments, one or more of the first or second chill plates are segmented into plural sub segments, sharing a common coolant pipe. In this method, the respective first and second inlets and outlets of the first and second chill plates are externally accessible outside of each respective DEA housing. The method includes orienting the plurality of DEAs within the gantry, so that their respective DEA housings are commonly coupled to a housing support of the gantry axially in sequence about an outside of the patient tunnel, parallel to a central axis defined by the patient tunnel; and that their respective detector elements face the patient tunnel, tangential to a circumference thereof, with components of the detector element projecting radially away from the patient tunnel. The method is further practiced by coupling the first inlet of the first chill plate of and the second inlet of the second chill plate of each DEA directly or indirectly to the coolant supply of the cooling apparatus; and coupling the first outlet of the first chill plate and the second outlet of the second chill plate of each DEA directly or indirectly to the coolant return of the cooling apparatus. Cooling capacity of the external cooling apparatus is scaled in proportion to the number of DEAs within the gantry that are cooled thereby. Coolant is circulated between the gantry and the cooling apparatus at a flow rate that maintains a specified stable temperature bandwidth for all detector elements in the each of the respective DEAs.

The respective features of the exemplary embodiments that are described herein may be applied jointly or severally in any combination or sub-combination.

The invention is defined in the appended claims.

### BRIEF DESCRIPTION OF DRAWINGS

The exemplary embodiments are further described in the following detailed description in conjunction with the accompanying drawings, in which:
FIG. 1 is a front elevational view of a gantry of a combination PET/CT medical imaging scanner for generating PET and/or CT images of a patient, which incorporates a circumferential and axial matrix array of modular, detector electronics assemblies (DEAs), sharing a common cooling system;
FIG. 2 is a schematic, side elevational cross section of a single, modular, DEA of the PET/CT scanner of FIG. 1;
FIG. 3 is a top plan view of the DEA of FIG. 2;
FIG. 4 is a schematic, elevational cross section of an axial row of four of the DEAs of FIG. 2 in the PET/CT scammer of FIG. 1, showing a cascading coolant flow loop of the cooling system;
FIG. 5 is a schematic, flat projection of a two-dimensional matrix grid of the circumferential array of the modular, DEAs of the medical imaging apparatus of FIG. 1, showing their cascading coolant flow loop of the cooling system among axial columns of the assemblies;
FIG. 6 is a schematic elevational view of detector arrays and a first detector chill plate of the DEA of FIG. 2:
FIG. 7 is a plan cross-sectional view of the first detector chill plate of FIG. 6;
FIG. 8 is an elevational cross-sectional view of the first detector chill plate of FIG. 6:
FIG. 9 is a side elevational view of an alternative embodiment of a chill plate;
FIG. 10 is a schematic of the cooling system of the PET/CT scanner of FIG. 1;
FIG. 11 is a plan view of a segmented first or second chill plate; and
FIG. 12 is a perspective view of a curved, segmented first or second chill plate, with cascading, parallel coolant flow between each sub segment thereof.

To facilitate understanding, identical reference numerals have been used, where possible, to designate identical elements that are common to the figures. The figures are not drawn to scale.

### DESCRIPTION OF EMBODIMENTS

Medical imaging apparatus with cooling systems incorporating cooling system embodiments described herein, in one or more of their modular, scalable detector electronics assemblies (DEAs), transfer heat out of system's gantry to maintain radiation detector and detector electronics within a designated temperature range, reducing the likelihood of temperature-related degradation of patient images. Various embodiments of these scalable, modular cooling systems are suitable for a broad range of axial field of view (aFOV) architecture applications, including computed tomography (CT), two-dimensional digital radiography (DR), positron emission tomography (PET), and single photon emission computed tomography (SPECT) modalities. Various embodiments of the cooling systems and their DEAs are also suitable for hybrid modality apparatuses that incorporate PET and another modality, (e.g., PET/CT or PET/MRI) of any length aFOV architecture.

Efficient cooling attributes of embodiments of these cooling systems are useful for DEAs that incorporate solid-state avalanche photo diodes (APDs) and silicon photomultipliers (SiPMs), as these types of solid-state detectors typically generate more operational heat than photo multiplier tubes (PMTs). Compared to PMTs, APDs and SiPMs are typically more susceptible to output signal distortion unless operated within relatively narrow temperature bandwidths. The scalable, modular cooling system embodiments described herein achieve high heat-load transfer out of the gantry of the imaging apparatus, facilitating operation of APDs and SiPMs within narrow temperature bandwidths, with less noise and construction complexity than known air-cooled systems.

The presently disclosed gantry cooling system embodiments transfer sufficient heat out of the gantry to maintain ambient operational temperature bandwidth and fluctuation specifications of the imaging system. More specifically, embodiments of the modular, scalable cooling systems disclosed herein efficiently transfer heat from the detector assembly of any modality of medical imaging apparatus by enhancing direct conductive heat transfer from radiation detectors, detector electronics and power supplies to gantry coolant in the cooling system. Cooling system components external the imaging system gantry dissipate heat from the gantry coolant. In this way, for specific aFOV length architectures, varying heat loads generated by varying numbers of DEAs are transferred out of the gantry by scaling the cooling system components located outside the gantry.

Some cooling system embodiments described herein facilitate fan-less direct water cooling of the electronics components, using thermal conductivity between the components and fluid-cooled, chill plate-type heat sinks as a heat transfer mode. Exemplary coolant fluids include compressible and incompressible fluids such as liquids and gases (e.g., room air, nitrogen, water) or phase-change refrigerants. The cooling system embodiments herein have overcome several design challenges. First, certain components such as the SiPM detector elements in the detector assemblies require lower and tighter temperature tolerances for the detectors to operate quantitatively within their design specifications. Second, electronics boards and other types of other electronic components associated with the detector assemblies, which also need to be cooled within the gantry, have irregular surface profiles and shapes that complicate capability of their direct contact with their associated, proximate, fluid-cooled heat sinks. Third, larger other electronic components in the DEA, such as power supplies are very bulky. Fourth, any fluid coolant leak within the gantry may cause electronics and power components therein to malfunction.

In various embodiments, modular, chill plate-type heat sinks are in direct contact with heat generating components. The chill plate has a fluid coolant line or conduit going through it with an inlet and an outlet. The plate material is selected for its thermal conductivity properties and may include one or more metals and/or thermally conductive ceramic compositions. Aluminum, and copper are typically used for the coolant lines. The coolant line takes several turns inside the plate to enhance heat transfer from plate to coolant. In some embodiments, the chill plate is designed as top and bottom plates with a groove for receiving the coolant line. Both plates are pressed against each other, sandwiching the coolant line therebetween, to enhance conductivity between the respective plate and its line. The term "chill plate", as used herein is intended to encompass solo, monolithic plates, as well as composite structures incorporating multiple subplates joined together to function as a unitary heat transfer medium, for absorbing heat generated or dissipated by an electronic component or other device within the associated DEA.

In some embodiments, the chill plates have a smooth external surface. In some embodiments, a thermal tape, or foam, or thermally conductive gel, or the like is interposed as a heat transfer median between the chill plate and its associated heat-generating detector elements, electronics board, or power supply. In other embodiments, the outer surface of the chill plate is fabricated to have the opposite, mirror image surface topography of its associated circuit board pattern, where the valleys in the chill plate profile encapsulate integrated circuit (IC) or other components on the circuit board that have high heat dissipation. In such embodiments, a thermally conductive material, such as thermal tape or foam, thermal grease or gel, or the like, is interposed as a thermal median between the plate surface and the associated component to aid the transfer of the heat from the component to the chill plate.

Embodiments of the cooling systems have scalable architecture, with one or more modular detector electronics assemblies (DEAs), allowing for axial FoV scalability of various imaging system configurations. Furthermore, having a DEA as a self-sufficient design in a housing with integrated input/output (I/O) communication of control/data information capability, electric power supply, chill plates with fluid coolant inlets and outlets, consolidates detector elements of detectors, electronics, and power supplies into one cohesive package. These DEA embodiments package the main heat generators/dissipators in the gantry of the imaging system, such as the detector electronics, other electronic component boards or printed circuit boards and power supplies into one package with its own dedicated cooling system components. In some embodiments a DEA's power supply is thermally coupled to a chill plate, to remove the heat from the former. By packaging other electronic components, such as the power supply, electronic boards, and printed circuit boards in the same housing as the detector elements, the integrated DEA is more compact, can share one or more chill plates among heat generating components, and minimize the number of coolant line connections between chill plates. In some DEA cooling system embodiments, a first chill plate is thermally coupled to a detector array of detector elements, and a second chill plate is thermally coupled to other electronic components, such as electronics board and a power supply. In some embodiments, the first chill plate is oriented within the DEA housing between the detector array of detector elements and the second chill plate, with the latter's associated other electronic components, such as electronics boards and power supplies.

In some embodiments the scalable cooling system is a closed loop system. This design has great advantages such as having a finite amount of coolant, such as water, that does not flood the system and facility if and where there is a leak, as compared to an open loop system with a relatively infinite coolant flow capability. This also allows the cooling system to provide stable coolant temperature to the chill plates within the DEAs, as ADP or SiPM components in their detector arrays require relatively narrow temperature bandwidth to operate quantitatively. In some imaging system embodiments, the specified coolant temperature bandwidth is 23°C with +/- 2°C to maintain a stable temperature to the SiPMs within the DEAs.

In some embodiments, coolant flowing to the gantry from the cooling apparatus is initially provided to the first chill plate associated with the detectors elements to maintain a tighter, stable temperature bandwidth on the SiPMs in the array. The output of the first chill plate is then plumbed to the second chill plate in the DEA, associated with the electronics and power supply. In some cooling system embodiments, where multiple DEAs are cascaded in the axial direction, the same flow sequence of coolant first flowing to the first chill plate in each DEA, then sequentially each second chill plate is maintained, for all DEAs in the cascade chain. Namely, coolant from the cooling apparatus first flows to the inlet for the first DEA in the cascade, then to the inlet of the second DEA in the cascade, and so on. In the last DEA in the cascade, its outflow from the outlet of its first chill plate is plumbed to its second chill plate. The cascade continues backwards, or upstream back to the return line of the cooling apparatus by routing coolant to and out of each successive second chill plate in the cascade, back to the first DEA in the chain. Thereafter, coolant discharged from the outlet of second chill plate of the first DEA is routed back to the cooling apparatus.

In some embodiments, to ensure all the DEAs in each axially interconnected column in the cooling system about the circumference of the gantry receive the same input coolant temperature, the coolant is branched out from the coolant supply to each DEA column via an annular ring manifold. In another alternative embodiment, coolant is branched out from the coolant supply to each interconnected DEA within an annular row or ring about the gantry circumference.

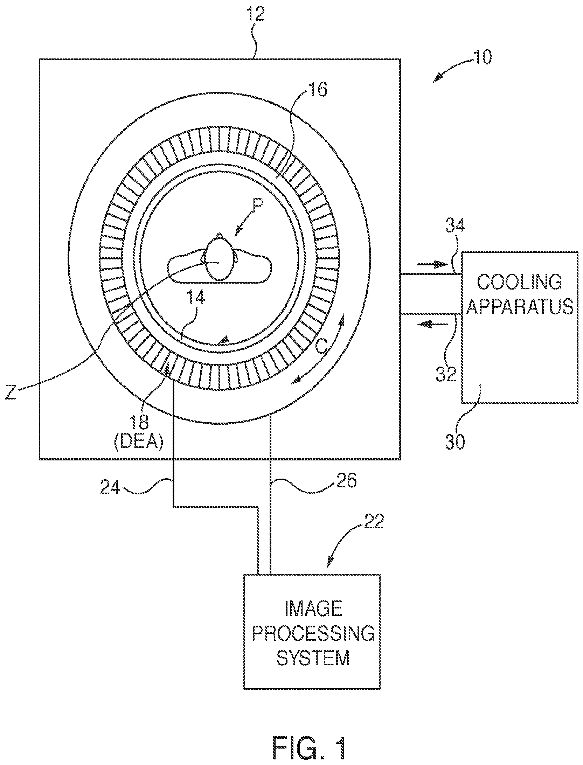

With reference to the figures, FIG. 1, shows a PET/CT imaging apparatus or system 10 for generating an overlaid PET and CT image display of a patient P. The apparatus 10 includes a gantry 12. A patient tunnel wall 14 in the gantry 12 defines an axial direction axis Z, extending orthogonally in relation to the plane of the drawing of FIG. 1. The patient tunnel wall 14 is circumscribed by an acoustic foam liner 16. A plurality of modular, detector electronics assemblies (DEAs) 18 are arranged coaxially in a matrix-like axial (Z direction) and circumferential (C directional arrow) array outside the patient tunnel wall 14 and the acoustic foam liner 16, equally radially spaced from the axis Z. An image processing system 22 is coupled to each DEA 18 by a communication and control signals pathway 24 and a power conduit 26. A cooling apparatus 30, oriented external the gantry 12 circulates fluid coolant through one or more of the DEAs 18, in a closed cooling loop, via coolant supply conduit 32 and fluid return conduit 34. Exemplary coolant fluids include compressible and incompressible fluids such as liquids and gases (e.g., room air, nitrogen, water) or phase-change refrigerants.

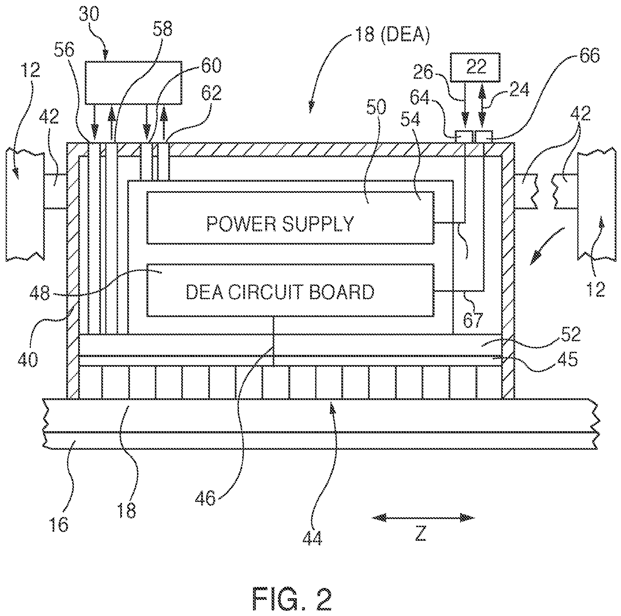

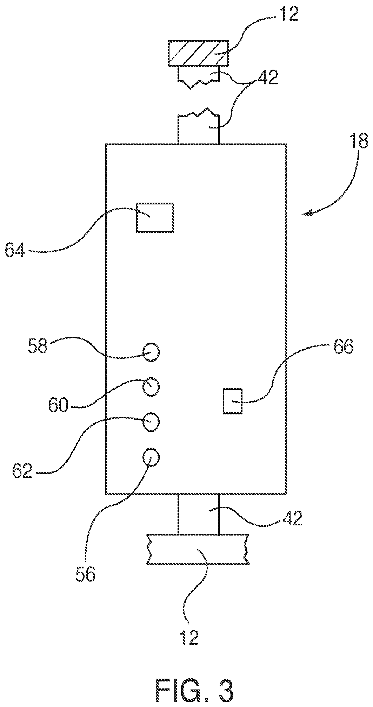

Referring to FIGs. 2 and 3, exemplary DEAs 18 comprise a modular DEA housing 40, coupled to the gantry 12 by a housing support 42. An exemplary DEA modular housing and housing support comprises a detector head, pivotable detector bearing and mounting rail as shown and described in co-pending United States Application No. 16/946,514. In jurisdictions permitting incorporation of patent documents by reference, the entire contents of Application No. 16/946,514. The DEA housing 40 includes a plurality of radiation detector elements 44, clustered in a two-dimensional array facing the patient P. Exemplary radiation detector elements 44 include avalanche photo diodes (APDs) or silicon photomultipliers (SiPMs). A detector data acquisition (DDA) package 45, for example incorporated on a printed circuit board, receives signals from the individual radiation detector elements 44 that are indicative of photons sensed by their detector crystals. The received detector crystal signals are routed from the DDA package 45, via a detector data-signal pathway 46 (e.g., a plug-in terminal-type, electrical connector), to a DEA electronic circuit board 48 for further signal processing. Some electronic circuit boards in the DEA incorporate printed circuit boards.

The DEA electronic circuit board 48 generates respective detector output signals, which are routed to the image processing unit 22, via a communications port 66 on the DEA housing 40 that interconnects an internal logic signals pathway 67 to the communication and control signals path 24. The detector output signals are subsequently processed by the image processing unit 22 to generate or construct patient images. In some embodiments, DDA package functionality is incorporated within the DEA circuit board rather than as a separate component. The DEA 18 also incorporates an internal DEA power supply 50. A power inlet 64 on the DEA housing 40 interconnects a power cable 65 of the power supply 50 to the external power conduit 26. Each DEA 18 is a self-contained modular unit, incorporating radiation detector elements 44, the electronics DDA 45, and related other electronics, (including by way of example the DEA electronics board or circuit board 48 to acquire and process signals indicative of incident photons sensed by the detectors, and routing output signals to the image processing unit 22, and the internal DEA power supply 50). Accordingly, any desired number of the individual, modular DEAs 18 are readily combined, by coupling each of its respective power inlet 64 and communications port 66 on the housing 40 into its respective complementary power conduit 26 and communication and control signals path 24 within the gantry 12, to create scalable two-dimensional matrices of detector elements 44 for any diameter and axial length patient tunnel wall dimensions within the gantry; including those of extended aFOV imaging apparatuses.

The modular DEA 18 also incorporates scalable cooling system architecture, complementary to the previously described scalable detector element 44 architecture. Varying heat transfer loads for different arrays of modular DEAs 18 are accommodated by altering the heat transfer capacity of the external cooling apparatus 30, rather than by altering internal structure of each DEA. The internal cooling system components in the DEA housing 40 comprise a fluid-cooled, first chill plate 52, oriented proximate the heat-generating radiation detector elements 44 and/or the DDA package 45, and a second chill plate, oriented proximate the heat-generating "other electronic components" (e.g., the DEA circuit board 48 and power supply 50).

In the embodiment of FIGs. 2-4, the first chill plate 52 is oriented generally parallel to the detector elements 44 and interposed between them and the relatively higher heat-generating "other electronic components" DEA circuit board 48 and power supply 50. This first chill plate 52 orientation provides additional heat shielding for the relatively more temperature sensitive detector elements 44. The second chill plate 54 is oriented generally perpendicular to and radially inwardly from the first chill plate 52, spanning almost the entire width and height of the DEA housing 40. This orientation of the second chill plate 54 provides a relatively large surface area and thermal mass for enhancing heat transfer from any other electronic components in the DEA, including by way of example the circuit board 48 and power supply 50. While the first 52 and second 54 chill plates shown in FIGs. 2-4 are generally planar, in other embodiments one or more of the chill plates, or sub-plate components thereof that are joined into a unitary, composite chill plate have non-planar profiles. Non-planar profile chill plate embodiments are suitable for wrapping around multiple surfaces of a heat generating/dissipating electronic component or other component.

To enhance direct, conductive heat transfer within the DEA 18, in some embodiments one or both of the first 52 and second 54 chill plates are at least partially in direct abutting, thermally conductive contact with their proximate heat-generating components. In other embodiments, one or both of the first 52 and second 54 chill plates are oriented in opposed, mutually spaced relationship with their proximate heat-generating components. To enhance conductive heat transfer across mutually spaced, opposed components, selectively, all or portions of the gaps therebetween are filled with a thermally conductive material median, such as thermally conductive sheet foam, tape, gel or grease.

Referring to FIGs. 1-3, the first 52 and second 54 chill plates transfer heat absorbed from their respective, proximate radiation detectors 44, DDA package 45, other electronic components, including the DEA circuit board 48, or power supply 50 to fluid coolant that is circulating within a closed loop between the gantry 12 and the external cooling apparatus 30. Within each DEA 18, the first chill plate 52 receives circulating fluid coolant entering through a first coolant inlet 56. Coolant flows through an internal conduit formed within the first chill plate 52, absorbing heat from the plate material by conductive and convective heat transfer modes and is discharged out of a first cooling outlet 58. Similarly, within each DEA 18, the second chill plate 54 receives circulating fluid coolant entering through a second coolant inlet 60. Coolant flows through an internal conduit formed within the second chill plate 54, absorbing heat from the plate material by conductive and convective heat transfer modes and is discharged out of a second cooling outlet 62.

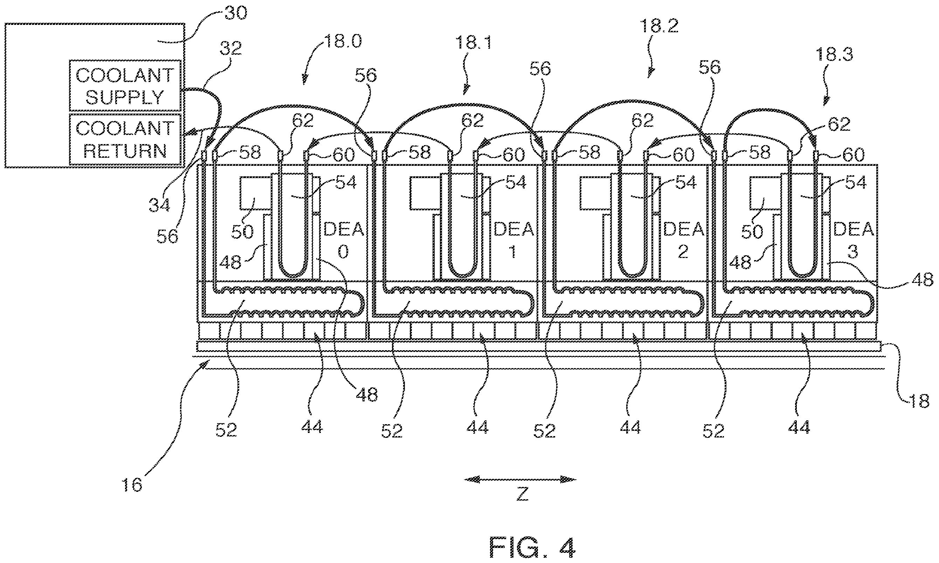

In some cooling system embodiments, respective inlets 56, 60 and outlets 58, 62 of the first 52 and second 54 chill plates communicate directly and independently with the cooling loop in the gantry 12, with parallel respective coolant flow paths for each DEA 18. In some medical imaging applications, it is desirable to minimize the number of coolant conduits in the gantry 12, to reduce likelihood of coolant leak damage to equipment. In some cooling system embodiments, providing cascading, serial coolant flow from the coolant supply conduit 32 of the cooling system 30, through the first chill plate 52, then the second chill plate 54 and back to the coolant return conduit 34 simplifies the cooling path and reduces the quantity of conduits, compared to cooling systems relying on independent, parallel coolant flow to each DEA. In other embodiments, the scalable, modular DEA 18 is used in a single component application (e.g., as a detector for a digital radiography imaging device). Referring to the DEA 18 shown in FIGs. 2 and 3, and the fluid flow path of DEA 18.3 of FIG. 4, in such single-component DEA applications, coolant from the coolant supply 32 of the cooling apparatus 30 enters the first coolant inlet 56 of the first chill plate 52, exits its first coolant outlet 58, enters the second coolant inlet 60 of the second chill plate 54 and exits its second coolant outlet 62, for discharge into the coolant return 34 of the cooling apparatus.

In the cooling system embodiment of FIG. 4, four DEAs, designated 18.0, 18.1, 18.2 and 18.3 are sequentially cooled in a cascading flow path, wherein DEA 18.0 is the first, most upstream DEA relative to the cooling apparatus 30 and DEA 18.3 is the last, most downstream DEA in the flow path. Components in each of the DEAs and coolant flow paths are shown schematically. All four of the respective first chill plates 52 receive coolant sequentially in a cascading fashion, in the direction of the flow arrows shown in the figure. The first DEA 18.0 in the cascading flow sequence receives flowing coolant from the coolant supply conduit 32 via the first inlet 56 of its first chill plate 52, which then exhausts the coolant via the first outlet 58. The coolant exhausted from the first outlet 58 of the first chill plate 52 of DEA 18.0 then enters DEA 18.1 via the first inlet 56 of its first chill plate 52, exhausting out of the latter's respective first outlet 58. Sequentially, coolant exhausting DEA 18.1 enters into and exhausts out of DEA 18.2, via the latter's first inlet 56 and first outlet 58 of its respective first chill plate 52. After exiting DEA 18.2, the coolant enters into the last, furthest downstream DEA 18.3 in the cascading fluid flow path, via the latter's first inlet 56 its respective first chill plate 52. By now, the first chill plate 52 in each and all of the respective DEAs 18.0-18.3 have been cooled.

Coolant returns to the cooling apparatus 30 by cascading flow through the second chill plates 54 of each of the respective DEAs in reverse order, from DEA 18.3 back to DEA 18.0 and the coolant return conduit 34, completing the coolant loop. Focusing now on the last downstream DEA 18.3 in the coolant flow path, coolant exhausted from the first outlet 58 of its first chill plate 52 flows into the second coolant inlet 60 of its second chill plate 54 and exhausts from the second coolant outlet 62. Thereafter, the coolant exiting DEA 18.3 flows back upstream toward the coolant return conduit 34 of the cooling apparatus 30, in cascading fashion, by entering the second coolant inlet 60 and exiting the second coolant outlet 62 of the second chill plate 54 of upstream DEA 18.2. Thereafter, in sequence, the coolant enters the second coolant inlet 60 and exits the second coolant outlet 62 of the second chill plate 54 of the next upstream DEA 18.1. Finally, the coolant enters the second coolant inlet 60 and exits the second coolant outlet 62 of the second chill plate 54 of the first upstream DEA 18.0, whereupon the now heated coolant returns and recirculates back to the cooling apparatus 30, via the coolant return conduit 34.

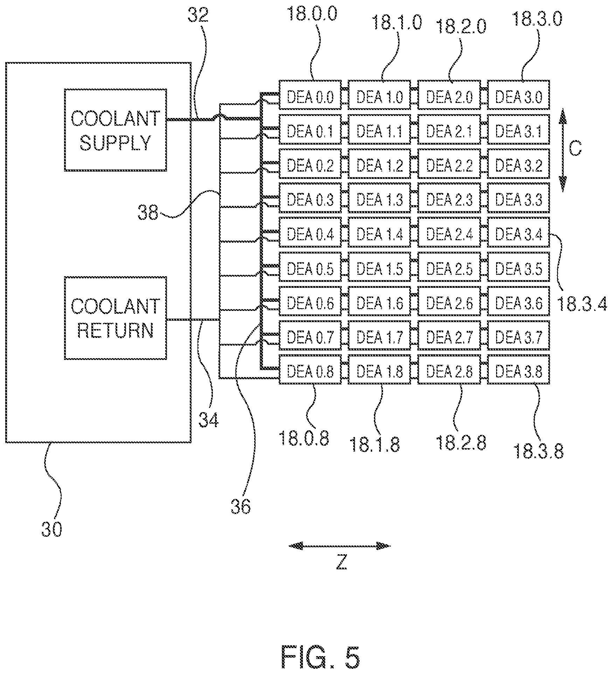

The described cascading, cooling flow path of FIG. 4 is scalable to accommodate any desired number of rows or columns of DEAs in the gantry 12. While four cascading DEAs 18.0, 18.1, 18.2 and 18.3, are shown in FIG. 4, other embodiments of cooling systems that incorporate the described cascading cooling flow path have fewer or greater numbers of DEAs. In some cooling system embodiments, the cascading cooling flow sequence of DEAs and their associated cooling system coolant conduits are selectively aligned in one or more columns, axially along the Z axis of the gantry 12, as shown in FIG. 5. The designated position of each DEA 18 in the matrix of detectors is DEA Δ.Θ, where Δ is the circumferential row in the directional axis C and Θ is the axial column in the directional axis Z. The bold line, coolant supply conduit 32 branches off, via an annular supply manifold 36 in parallel to each of the columns, designated DEA Δ.0 to Δ.8, feeding coolant, in cascading fashion, to each respective downstream, first chill plate in the associated column. Coolant exiting the first outlet of each respective, first chill plate of the last downstream DEA 3.0 to 3.8 in turn is routed to the second inlet of its corresponding respective second chill plate, and sequentially back upstream, from its respective second outlet to the outlet of the next upstream corresponding DEA 2.0 to 2.8, thereafter upstream to corresponding DEA 1.0 to 1.8, and lastly to the first upstream corresponding DEA 0.0 to 0.8. Coolant exiting the corresponding second outlet of the second chill plate in DEAs 0.0 to 0.8 flows in parallel, via annular return manifold 38 back to the coolant return 34 of the coolant system 30. In other cooling system embodiments, the cascading coolant flow path from the coolant supply 32 to the coolant return 34 of the cooling system 30 is parallel among the circumferential rows or rings of DEAs 0.Θ to 3. Θ (not shown in the figures).

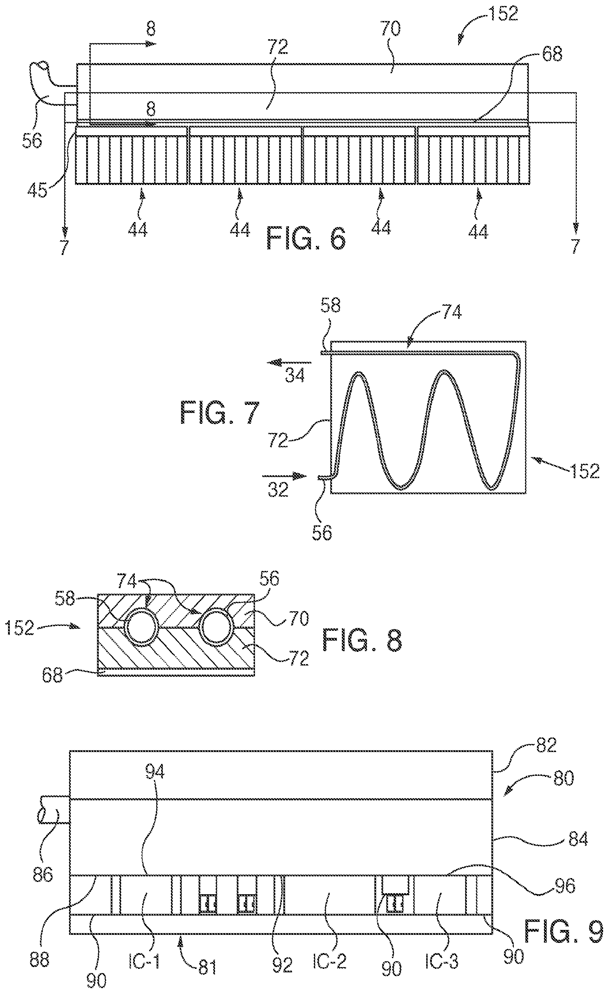

FIGs. 6-8 show another exemplary embodiment of a first chill plate 152, wherein a thermally conductive foam layer 68 fills gaps or voids between that chill plate and its corresponding, opposing detector element components of the detector elements 44 and DDA 45. In other embodiments, other thermally conductive material, such as thermally conductive tape, thermal gel and/or thermal grease substitutes for or is used in conjunction with the thermal foam layer 68. The first chill plate 152 is a split-construction, joined composite of a top plate 70 and a bottom plate 72. A coolant pipe 74 forms the first coolant inlet 56 and the first coolant outlet 58; it is in thermally conductive communication with and sandwiched between the thermally conductive top 70 and bottom 72 plates. Inwardly facing, opposing surfaces of the top 70 and bottom 72 plates conform to the outer surface profile of the corresponding coolant pipe 74, for direct contact therebetween, or with any gaps or voids filled with thermally conductive material. In various embodiments, the conforming profiles of inwardly facing, opposing surfaces of the top 70 and bottom 72 plates are formed by known casting, molding, 3-D printing and/or machining manufacturing process. In some embodiments, the first chill plate 52 is cast or molded in place about the coolant pipe 74, without the need for a sandwiched construction with joined separate top 70 and bottom 72 plates. In plan form, such as the serpentine-axial profile shown in FIG. 7, the coolant pipe 74 is formed in any desired axial profile that enhances conductive heat transfer from the top 70 and bottom 72 plates to coolant flowing through the pipe. The chill plate 52, including its top 70 and bottom 72 plates and its coolant pipe 74 are constructed of thermally conductive material. In some embodiments, the chill plate is constructed as shown and described as a cooling channel and heat sink in co-pending International Application No. PCT/US2020/070462.

FIG. 9 is another embodiment of a chill plate 80 for absorbing heat generated by a proximate circuit board 81. The chill plate 80 has a sandwiched construction like that of the first chill plate 152 of FIGs. 6-8. The opposing joined second top plate 82 and second bottom plate 84 capture a second coolant pipe 86 therebetween. To enhance conductive heat transfer from the opposing circuit board 81, one or more regions of the bottom face 88 of the second bottom plate 84 have formed elevational surface profiles that are mirror images of the elevational surface profile of a corresponding region of the circuit board. In some regions, the bottom face 88 forms a post or island 90 projecting outwardly therefrom, towards the circuit board 81. In other regions the bottom face 88 forms a well or cavity 92 that receives a component, such as the integrated circuit packages IC-2 and IC-3 projecting from the circuit board 81. At the region 94 of the bottom face 88, the integrated circuit package IC-1 is in direct abutting contact with the chill plate 80. At the region 96, the integrated circuit package IC-3 is spaced away from the opposing bottom face 88; the gap therebetween is filled with thermally conductive grease or gel, or any other desired thermally conductive filler material.

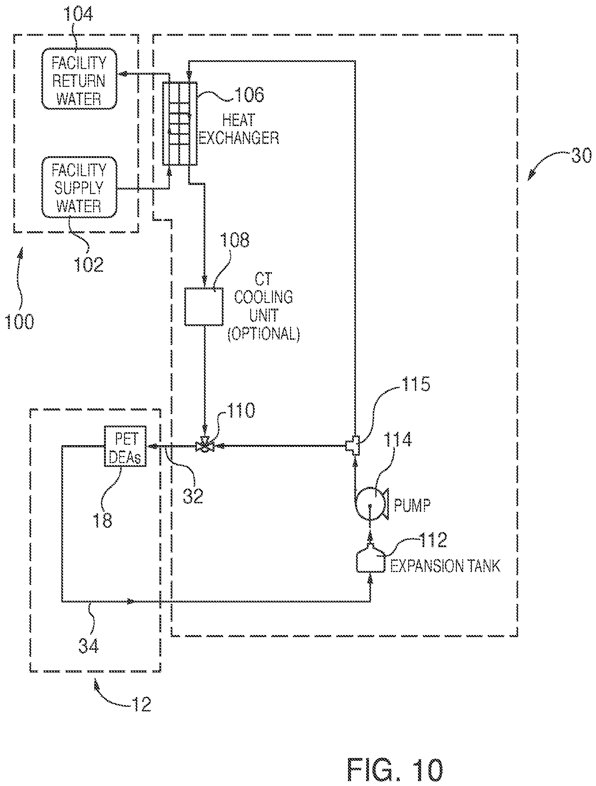

FIG. 10 is a schematic of an exemplary embodiment of a cooling apparatus 30 of the extended aFOV, PET/CT modality, medical imaging apparatus 10, comprising a fluid, continuous flow, closed coolant loop, circulating a liquid coolant such as water or a compressed, gaseous coolant, such as nitrogen or compressed air. In other embodiments, the coolant is a mixed phase, liquid/gas refrigerant, in which case the cooling apparatus incorporates a refrigeration system with an expansion valve, compressor, condenser and the like (not shown). In general, flowing coolant in the coolant loop absorbs heat generated by various components within the gantry 12, such as the exemplary DEA 18, and transfers the absorbed heat to a heat sink.

Here, the heat sink is a facility water system 100, which provides a flowing, cool water supply 102 into an intake loop of a liquid-liquid heat exchanger 106; thereafter, warmer return water 104 exits the heat exchanger. The previously circulated, heated coolant from the gantry 12 flows through a corresponding outlet loop of the heat exchanger 106, transferring heat to return water 104. In some embodiments, coolant exiting the heat exchanger 106 passes through an optional CT cooling unit 108 before entering a tee-type mixing valve 110.

The mixing valve 110 selectively mixes proportionally cooled coolant that has passed through the heat exchanger 106 and relatively hotter coolant from the coolant return conduit 34 to achieve a desired coolant temperature. Coolant of the desired coolant temperature exits the mixing valve 110 into the coolant supply conduit 32, where it enters the gantry 12, absorbs heat from the DEAs 18 and any other, if any, cooled components in the gantry. More specifically, the coolant in the coolant supply conduit 32 passes through the previously described first and second chill plates of one or more of the DEAs 18, where it absorbs heat generated by various internal detector elements, circuit electronic boards and power supplies, etc. The now heated coolant returns to the cooling apparatus 30 via the coolant return conduit 34. The heated coolant received from the coolant return conduit 34 is stored in an expansion tank 112. Circulating pump 114 pumps the still heated coolant through the coolant loop through bypass tee 116, where a portion of the heated coolant flows to the heat exchanger 106, for subsequent refresh cooling and the remaining portion of the heated coolant is routed to the mixing valve 110. The mixing valve 110 and the pump 114 adjust flow rate and mixing proportions of the recirculating coolant to achieve desired heat absorption from the gantry. One specific coolant temperature control parameter of interest is maintaining a stable temperature bandwidth of the detector elements in each DEA within specification parameters, to avoid detector distortion. In some imaging system embodiments, where its DEAs incorporate SiPM detector elements, the coolant temperature bandwidth is 23°C within +/- 2°C. The mixing valve 110 and the pump 114 circulate the coolant between the gantry 12 and the cooling apparatus 30 at a flow rate that maintains a specified stable temperature bandwidth for all detector elements in the each of the respective DEAs 18 in the gantry.

Heat absorption and transfer capacity of the external cooling apparatus 30 is proportionately scaled to the number of modular DEAs 18 in the gantry 12. As each modular DEA 18 incorporates its own dedicated internal cooling components (e.g., its first and second chill plates and their related coolant inlets and outlets), there is no need to add additional configurations of auxiliary cooling devices, such as cooling fans, to the gantry 12, when changing the number of DEAs in the gantry for different imaging scanning field dimensions.

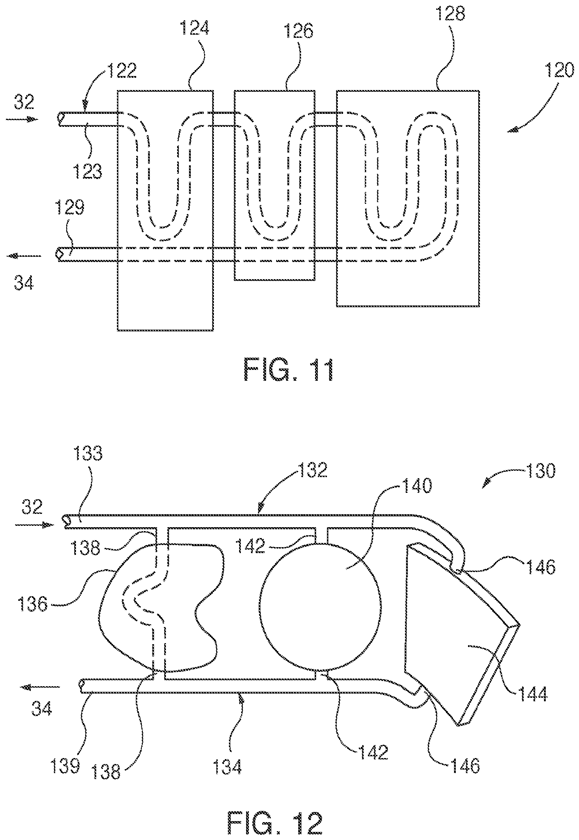

FIGs. 11 and 12 are embodiments of segmented chill plates that incorporate plate sub-segments with commonly shared inlets and outlets. Each sub-segment functions as an independent chill plate. Segmented chill plates are useful for packaging and distributing heat absorption and heat isolation capacities into smaller, confined zones within a DEA that cannot physically accommodate a single, larger chill plate having the same heat absorption capacity. They are also useful for conforming to multiple faces of an electronic component, such as a base and one or more lateral sides of a power supply. In other applications, segmented chill plates provide selective heat isolation between components within a DEA housing.

The segmented chill plate 120 incorporates cascading, sequential coolant flow from coolant supply 32 to coolant return 34, via a continuous coolant pipe 122, to each of the respective first 124, second 126 and third 128 chill plate sub-segments. The coolant pipe 122 captured within the chill plate sub-segments has a serpentine profile, similar to the coolant pipe 74 of FIG. 7. In this embodiment of FIG. 11, each of those sub-segments124, 126, 128 have differing surface area profiles. The coolant inlet side 123 of the coolant pipe 122 is in direct or indirect communication with the coolant supply 32 and the coolant outlet side 129 thereof is in direct or indirect communication with the coolant return 34. Conceptually in some embodiments, the segmented chill plate 120 is readily substituted for any one of the first 52 or second 54 chill plates shown in any of the DEAs of FIGs. 2 or 4. For example, if the segmented chill plate 120 is substituted for any one of the second chill plates 54 of FIG. 4, its coolant inlet 123 substitutes for the second coolant inlet 60 and its coolant outlet 129 substitutes for the second coolant outlet 62.

The curved, segmented chill plate 130 of FIG. 12 incorporates parallel coolant flow architecture, with plate coolant-supply manifold 132, whose inlet 133 is in direct or indirect communication with the coolant supply 32, and plate coolant-return manifold 134, whose outlet 135 is in direct or indirect communication with the coolant return 34. In this exemplary coolant flow architecture, a first chill plate sub-segment 136 incorporates a first coolant pipe 138, a second chill plate sub-segment 140 incorporates a second coolant pipe 142 and a third chill plate sub-segment 144 incorporates a third coolant pipe 146. The opposite ends of the first 138, second 140 and third 144 coolant pipes are respectively in fluid communication with the coolant- supply manifold 132 and the coolant-return manifold 134. Each chill plate sub-segment has a different planform profile for conductive heat absorption from a corresponding heat dissipating component within a DEA. The third chill plate sub-segment 144 has a curved planform profile.

The modular, scalable, fluid cooling systems described herein operate within imaging system gantries at lower noise levels than existing forced air, with air-liquid heat exchanger-type cooling systems. In an exemplary modular, fluid cooling system embodiment, designed for incorporation within a long aFOV PET system, such as shown in FIGs. 1 and 10, operating noise within the patient tunnel was measured to be less than fifty-five decibels (55 dB). As shown in FIG. 10, coolant pumps and other noise generating components are in the external cooling apparatus 30 outside the gantry 12. The fluid cooling system embodiments described herein do not need or utilize noisy cooling fans within the gantry 12. Additionally, the acoustic foam liner 16 circumscribing the patient tunnel wall 14 further suppresses noise within the patient tunnel. In contrast, in a comparable long aFOV PET system, employing a forced air-cooling system, the measured noise level in its patient tunnel was on the order greater than ten decibels (> 10dB) higher than that of the exemplary PET system with the modular, fluid cooling system.

Scalable cooling system embodiments disclosed herein maintain thermal operating stability of detector elements, such as SiPMs, no matter how many modular DEAs are ganged together. For example, in a long aFOV PET/CT system, four gantries incorporating the disclosed DEAs are ganged together axially, with the previously described cascading coolant flow between the first chill plates in each DEA, followed by cascading flow through the second chill plates. The cascading cooling flow interconnection reduces the number of coolant fittings and coolant lines within the gantry or gantries. All the interconnected DEAs are desirably serviced by a single coolant pump of the cooling apparatus. The modular DEAs are compact, reducing needed gantry internal volume, despite increasing axial lengths of long aFOV imaging systems.

Although various embodiments have been shown and described in detail herein, others can readily devise many other varied embodiments that still incorporate the claimed invention. The invention is not limited in its application to the exemplary embodiment details of construction and the arrangement of components set forth in the description or illustrated in the drawings. The invention is capable of other embodiments and of being practiced or of being carried out in various ways. In addition, it is to be understood that the phraseology and terminology used herein is for the purpose of description and should not be regarded as limiting. The use of "including", "comprising", or "having", and variations thereof herein is meant to encompass the items listed thereafter and equivalents thereof as well as additional items. Unless specified or limited otherwise, the terms '"mounted", "connected", "supported", and "coupled" and variations thereof are to be interpreted broadly; they encompass direct and indirect mountings, connections, supports, and couplings.

## Claims

1. A fluid coolant system for of a medical imaging apparatus (10), comprising:
a gantry (12) forming a patient tunnel;
a cooling apparatus (30), coupled to and external the gantry (12), having a coolant supply for supplying fluid coolant to the gantry (12), and a coolant return for returning the coolant to the cooling apparatus (30);
a detector electronic assembly (DEA) (18) within the gantry (12), coupled to the cooling apparatus (30), having:
a housing (40);
detector elements (44) in the housing (40), for detecting incident photons of electromagnetic radiation originating outside of the housing (40);
other electronic components (48, 50) in the housing (40);
a fluid cooled, first chill plate (52, 152) thermally conductively coupled to the detector elements (44), for cooling the detector elements (44), the first chill plate (52, 152) having a first inlet (56) for receiving the coolant from the coolant supply and a first outlet (58) for discharging the coolant to the coolant return; and
a fluid cooled, second chill plate (54) thermally conductively coupled to the other electronic components (48, 50) for cooling the other electronic components (48, 50), the second chill plate (54) having a second inlet (60) for receiving the coolant from the coolant supply and a second outlet (62) for discharging the coolant to the coolant return;
the fluid coolant system, further comprising:
the detector elements (44) having detector element components projecting away therefrom, defining a first surface profile;
the first chill plate (52, 152) in thermally conductive, direct contact with at least a portion of the first surface profile of the detector element components;
at least one of the other electronic components (48, 50) defining a second surface profile; and
the second chill plate (54) in thermally conductive, direct contact with at least a portion of the second surface profile of the at least one of the other electronic components (48, 50).

2. The fluid coolant system of claim 1, further comprising:
the detector elements (44) in the DEA (18) facing the patient tunnel, the detector elements (44) having detector element components projecting radially away from the patient tunnel;
the first chill plate (52, 152) in thermally conductive, direct contact with at least one of the detector element components and oriented in the housing (40) intermediate the detector element components and the other electronic components (48, 50); and
the second chill plate (54) in thermally conductive, direct contact with the other electronic components (48, 50).

3. A detector electronic assembly (DEA) (18) for a medical imaging apparatus (10), comprising:
a housing (40);
detector elements (44) retained in the housing (40), for detecting incident photons of electromagnetic radiation originating outside of the housing (40);
other electronic components (48, 50) in the housing (40);
a fluid cooled, first chill plate (52, 152) thermally conductively coupled to the detector elements (44), for cooling the detector elements (44), the first chill plate (52, 152) having a first inlet (56) for receiving fluid coolant and a first outlet (58) for discharging the coolant;
a fluid cooled, second chill plate (54) thermally conductively coupled to the other electronic components (48, 50), for cooling the other electronic components (48, 50), the second chill plate (54) having a second inlet (60) for receiving the coolant and a second outlet (62) for discharging the coolant;
the respective first and second inlets (60) and outlets accessible outside the housing (40);
the DEA (18), further comprising:
the detector elements (44) having detector element components projecting away therefrom, defining a first surface profile;
the first chill plate (52, 152) in thermally conductive, direct contact with at least a portion of the first surface profile of the detector element components;
at least one of the other electronic components (48, 50) defining a second surface profile; and
the second chill plate (54) in thermally conductive, direct contact with at least a portion of the second surface profile of the at least one of the other electronic components (48, 50).

4. The fluid coolant system of claim 1 or the DEA (18) of claim 3, further comprising:
the first chill plate (52, 152) defining a first surface profile that is a mirror image of the first surface profile of the detector elements (44), the respective first surface profiles in thermally conductive, direct contact with each other; and
the second chill plate (54) defining a second surface profile that is a mirror image of the second surface profile of the at least one of the other electronic components (48, 50), the respective second surface profiles in thermally conductive, direct contact with each other.

5. The fluid coolant system of claim 2 or the DEA (18) of claim 4, further comprising the first outlet (58) of the first chill plate (52, 152) in fluid communication with the second inlet (60) of the second chill plate (54), so that coolant first flows through the first chill plate (52, 152) and then sequentially in cascading fashion flows through the second chill plate (54).

6. The DEA (18) of claim 3, further comprising:
the detector elements (44) in the DEA (18) are exposed outside of the housing (40), with the detector element components projecting into the housing (40);
the first chill plate (52, 152) in thermally conductive, direct contact with at least one of the detector element components and oriented in the housing (40) intermediate the detector element components and the other electronic components (48, 50); and
the second chill plate (54) in thermally conductive, direct contact with the other electronic components (48, 50).

7. The fluid coolant system of claim 5 or the DEA (18) of claim 6, further comprising:
the second chill plate (54) having opposed first and second sides; and
the other electronic components (48, 50) including a printed circuit board and a power supply respectively thermally coupled to the respective first and second sides of the second chill plate (54).

8. The DEA (18) of claim 3, further comprising:
the second chill plate (54) having opposed first and second sides; and
the other electronic components (48, 50) including a printed circuit board and a power supply respectively thermally coupled to the respective first and second sides of the second chill plate (54).

9. The DEA (18) of claim 3, further comprising a thermally conductive material interposed between the first chill plate (52, 152) and its thermally conductively coupled detector elements (44) and/or between the second chill plate (54) and its and its thermally conductively coupled other electronic components (48, 50).

10. The DEA (18) of claim 3, further comprising at least one of the first or the second chill plates (54) respectively having:
a coolant pipe with a first end forming the respective first or second inlet (60) and a second end forming the respective first or second outlet (62); and
split construction respective top and bottom plates in direct contact with and capturing the coolant pipe therebetween.

11. The fluid coolant system of claim 1 or the DEA (18) of claim 3, the first chill plate (52, 152) or the second chill plate (54) further comprising plate sub-segments with commonly shared, respective first or second inlets (60) and first or second outlets (62).

12. A medical imaging system incorporating a plurality of the DEAs (18) of claim 3, further comprising:
a cooling apparatus (30), coupled to and external the gantry (12), having a coolant supply for supplying fluid coolant to the gantry (12), and a coolant return for returning the coolant to the cooling apparatus (30);
the plurality of DEAs (18) coupled to the cooling apparatus (30);
the respective DEA (18) housings (40) commonly coupled to a housing (40) support of a gantry (12) of the medical imaging system, the DEA (18) housings (40) oriented axially in sequence about an outside of a patient tunnel of the gantry (12), parallel to a central axis defined by the patient tunnel;
the respective detector elements (44) in of the DEAs (18) facing the patient tunnel, oriented outside of and tangential to a circumference of the patient tunnel, with detector element components projecting radially away from the patient tunnel;
the first chill plate (52, 152) in each of the respective DEAs (18) in thermally conductive, direct contact with at least one of the detector element components and oriented in the housing (40) intermediate the detector element components and the other electronic components (48, 50);
the second chill plate (54) in each of the respective DEAs (18) having opposed first and second sides oriented normal to the circumference of the patient tunnel;
the other electronic components (48, 50) including a printed circuit board and a power supply, respectively thermally coupled to the respective first and second sides of the second chill plate (54); and
the respective first inlets (56) of all of the first chill plates (52, 152) of the DEAs (18) collectively receiving coolant from the coolant supply and subsequently discharging the coolant out of their respective first outlets (58), whereupon the discharged coolant is subsequently collectively received in the respective second inlets (60) of all of the second chill plates (54) and then discharged out of their respective second outlets (62) to the coolant return.

13. A method for scalable cooling of a gantry (12) of a medical imaging apparatus (10), comprising:
providing a gantry (12) forming a patient tunnel;
providing a cooling apparatus (30), coupled to and external the gantry (12), having a coolant supply for supplying fluid coolant to the gantry (12), and a coolant return for returning the coolant to the cooling apparatus (30);
providing a selected plurality of detector electronic assemblies (DEAs) (18) for orientation within the gantry (12), each respective DEA (18) having:
a housing (40);
detector elements (44) in the housing (40), for detecting incident photons of electromagnetic radiation originating outside of the housing (40);
other electronic components (48, 50) in the housing (40), including a printed circuit board and a power supply;
a fluid cooled, first chill plate (52, 152) thermally conductively coupled to the detector elements (44), for cooling the detector elements (44) and oriented in the housing (40) intermediate the detector elements (44) and the other electronic components (48, 50), the first chill plate (52, 152) having a first inlet (56) for receiving the coolant from the coolant supply and a first outlet (58) for discharging the coolant to the coolant return; and
a fluid cooled, second chill plate (54) for cooling the other electronic components (48, 50), the second chill plate (54) having opposed first and second sides, respectively thermally conductively coupled to the printed circuit board on its first side and to the power supply on its second side, the second chill plate (54) having a second inlet (60) for receiving the coolant from the coolant supply and a second outlet (62) for discharging the coolant to the coolant return;
the respective first and second inlets (60) and outlets of the first and second chill plates (54) externally accessible outside of each respective DEA (18) housing (40);
orienting the plurality of DEAs (18) within the gantry (12), so that:
their respective DEA (18) housings (40) are commonly coupled to a housing (40) support of the gantry (12) axially in sequence about an outside of the patient tunnel, parallel to a central axis defined by the patient tunnel; and
their respective detector elements (44) face the patient tunnel, tangential to a circumference thereof, with components of the detector element projecting radially away from the patient tunnel;
coupling the first inlet (56) of the first chill plate (52, 152) of and the second inlet (60) of the second chill plate (54) of each DEA (18) directly or indirectly to the coolant supply of the cooling apparatus (30);
coupling the first outlet (58) of the first chill plate (52, 152) and the second outlet (62) of the second chill plate (54) of each DEA (18) directly or indirectly to the coolant return of the cooling apparatus (30);
scaling cooling capacity of the external cooling apparatus (30) in proportion to the number of DEAs (18) within the gantry (12) that are cooled thereby; and
circulating the coolant between the gantry (12) and the cooling apparatus (30) at a flow rate that maintains a specified stable temperature bandwidth for all detector elements (44) in the each of the respective DEAs (18).

14. The method for scalable cooling of a gantry (12) of claim 13, further comprising:
collectively coupling all of the first inlets (56) of the first chill plates (52, 152) to the coolant supply of the cooling apparatus (30); and
collectively coupling all of the second inlets (60) of the second chill plates (54) to the collective first outlets (58) of the first chill plates (52, 152), so that coolant from the coolant supply first flows through all of the first chill plates (52, 152) prior flowing through the second chill plates (54).

15. The method for scalable cooling of a gantry (12) of claim 13, the first chill plate (52, 152) or the second chill plate (54) further comprising plate sub-segments with commonly shared, respective first or second inlets (60) and first or second outlets (62).

## Patentansprüche

1. Fluidkühlmittelsystem für eine medizinische Bildgebungseinrichtung (10), umfassend:
eine Gantry (12), die einen Patiententunnel bildet;
eine Kühleinrichtung (30), die mit und extern zur Gantry (12) gekoppelt ist und eine Kühlmittelzufuhr zum Zuführen von fluidem Kühlmittel zur Gantry (12) und eine Kühlmittelrückführung zum Zurückführen des Kühlmittels zur Kühleinrichtung (30) aufweist;
eine elektronische Detektorbaugruppe (DEA) (18) innerhalb der Gantry (12), die mit der Kühleinrichtung (30) gekoppelt ist und aufweist:
ein Gehäuse (40);
Detektorelemente (44) im Gehäuse (40) zum Detektieren von einfallenden Photonen von elektromagnetischer Strahlung, die von außerhalb des Gehäuses (40) herrührt;
andere elektronische Komponenten (48, 50) im Gehäuse (40);
eine fluidgekühlte erste Kühlplatte (52, 152), die thermisch leitend mit den Detektorelementen (44) gekoppelt ist, zum Kühlen der Detektorelemente (44), wobei die erste Kühlplatte (52, 152) einen ersten Einlass (56) zum Aufnehmen des Kühlmittels von der Kühlmittelzufuhr und einen ersten Auslass (58) zum Ablassen des Kühlmittels zur Kühlmittelrückführung aufweist; und
eine fluidgekühlte zweite Kühlplatte (54), die thermisch leitend mit den anderen elektronischen Komponenten (48, 50) gekoppelt ist, zum Kühlen der anderen elektronischen Komponenten (48, 50), wobei die zweite Kühlplatte (54) einen zweiten Einlass (60) zum Aufnehmen des Kühlmittels von der Kühlmittelzufuhr und einen zweiten Auslass (62) zum Ablassen des Kühlmittels zur Kühlmittelrückführung aufweist;
wobei das Fluidkühlmittelsystem ferner umfasst:
die Detektorelemente (44), die Detektorelementkomponenten aufweisen, die davon wegragen und ein erstes Oberflächenprofil definieren;
die erste Kühlplatte (52, 152) in thermisch leitendem, direktem Kontakt mit zumindest einem Abschnitt des ersten Oberflächenprofils der Detektorelementkomponenten;
mindestens eine der anderen elektronischen Komponenten (48, 50), die ein zweites Oberflächenprofil definiert; und
die zweite Kühlplatte (54) in thermisch leitendem, direktem Kontakt mit zumindest einem Abschnitt des zweiten Oberflächenprofils der mindestens einen der anderen elektronischen Komponenten (48, 50).

2. Fluidkühlmittelsystem nach Anspruch 1, ferner umfassend:
die Detektorelemente (44) in der DEA (18), die dem Patiententunnel zugewandt sind, wobei die Detektorelemente (44) Detektorelementkomponenten aufweisen, die radial vom Patiententunnel wegragen;
die erste Kühlplatte (52, 152) in thermisch leitendem, direktem Kontakt mit mindestens einer der Detektorelementkomponenten und im Gehäuse (40) zwischen den Detektorelementkomponenten und den anderen elektronischen Komponenten (48, 50) ausgerichtet; und
die zweite Kühlplatte (54) in thermisch leitendem, direktem Kontakt mit den anderen elektronischen Komponenten (48, 50).

3. Elektronische Detektorbaugruppe (DEA) (18) für eine medizinische Bildgebungseinrichtung (10), umfassend:
ein Gehäuse (40);
Detektorelemente (44), die im Gehäuse (40) untergebracht sind, zum Detektieren von einfallenden Photonen von elektromagnetischer Strahlung, die von außerhalb des Gehäuses (40) herrührt;
andere elektronische Komponenten (48, 50) im Gehäuse (40);
eine fluidgekühlte erste Kühlplatte (52, 152), die thermisch leitend mit den Detektorelementen (44) gekoppelt ist, zum Kühlen der Detektorelemente (44), wobei die erste Kühlplatte (52, 152) einen ersten Einlass (56) zum Aufnehmen von fluidem Kühlmittel und einen ersten Auslass (58) zum Ablassen des Kühlmittels aufweist;
eine fluidgekühlte zweite Kühlplatte (54), die thermisch leitend mit den anderen elektronischen Komponenten (48, 50) gekoppelt ist, zum Kühlen der anderen elektronischen Komponenten (48, 50), wobei die zweite Kühlplatte (54) einen zweiten Einlass (60) zum Aufnehmen des Kühlmittels und einen zweiten Auslass (62) zum Ablassen des Kühlmittels aufweist;
wobei die jeweiligen ersten und zweiten Einlässe (60) und Auslässe außerhalb des Gehäuses (40) zugänglich sind;
wobei die DEA (18) ferner umfasst:
die Detektorelemente (44), die Detektorelementkomponenten aufweisen, die davon wegragen und ein erstes Oberflächenprofil definieren;
die erste Kühlplatte (52, 152) in thermisch leitendem, direktem Kontakt mit zumindest einem Abschnitt des ersten Oberflächenprofils der Detektorelementkomponenten;
mindestens eine der anderen elektronischen Komponenten (48, 50), die ein zweites Oberflächenprofil definiert; und
die zweite Kühlplatte (54) in thermisch leitendem, direktem Kontakt mit zumindest einem Abschnitt des zweiten Oberflächenprofils der mindestens einen der anderen elektronischen Komponenten (48, 50).

4. Fluidkühlmittelsystem nach Anspruch 1 oder DEA (18) nach Anspruch 3, ferner umfassend:
die erste Kühlplatte (52, 152), die ein erstes Oberflächenprofil definiert, das ein Spiegelbild des ersten Oberflächenprofils der Detektorelemente (44) ist, wobei die jeweiligen ersten Oberflächenprofile in thermisch leitendem, direktem Kontakt miteinander stehen; und
die zweite Kühlplatte (54), die ein zweites Oberflächenprofil definiert, das ein Spiegelbild des zweiten Oberflächenprofils der mindestens einen der anderen elektronischen Komponenten (48, 50) ist, wobei die jeweiligen zweiten Oberflächenprofile in thermisch leitendem, direktem Kontakt miteinander stehen.

5. Fluidkühlmittelsystem nach Anspruch 2 oder DEA (18) nach Anspruch 4, ferner umfassend den ersten Auslass (58) der ersten Kühlplatte (52, 152) in Fluidkommunikation mit dem zweiten Einlass (60) der zweiten Kühlplatte (54), sodass Kühlmittel zunächst durch die erste Kühlplatte (52, 152) fließt und dann folgend kaskadierend durch die zweite Kühlplatte (54) fließt.

6. DEA (18) nach Anspruch 3, ferner umfassend:
die Detektorelemente (44) in der DEA (18), die außerhalb des Gehäuses (40) freigelegt sind, wobei die Detektorelementkomponenten in das Gehäuse (40) hineinragen;
die erste Kühlplatte (52, 152) in thermisch leitendem, direktem Kontakt mit mindestens einer der Detektorelementkomponenten und im Gehäuse (40) zwischen den Detektorelementkomponenten und den anderen elektronischen Komponenten (48, 50) ausgerichtet; und
die zweite Kühlplatte (54) in thermisch leitendem, direktem Kontakt mit den anderen elektronischen Komponenten (48, 50).

7. Fluidkühlmittelsystem nach Anspruch 5 oder DEA (18) nach Anspruch 6, ferner umfassend:
die zweite Kühlplatte (54), die eine gegenüberliegende erste und zweite Seite aufweist; und
die anderen elektronischen Komponenten (48, 50), die eine Leiterplatte und eine Leistungsversorgung aufweisen, die jeweils thermisch mit der jeweiligen ersten und zweiten Seite der zweiten Kühlplatte (54) gekoppelt sind.

8. DEA (18) nach Anspruch 3, ferner umfassend:
die zweite Kühlplatte (54), die eine gegenüberliegende erste und zweite Seite aufweist; und
die anderen elektronischen Komponenten (48, 50), die eine Leiterplatte und eine Leistungsversorgung aufweisen, die jeweils thermisch mit der jeweiligen ersten und zweiten Seite der zweiten Kühlplatte (54) gekoppelt sind.

9. DEA (18) nach Anspruch 3, ferner umfassend ein thermisch leitendes Material, das zwischen der ersten Kühlplatte (52, 152) und ihren thermisch leitend gekoppelten Detektorelementen (44) und/oder zwischen der zweiten Kühlplatte (54) und ihren thermisch leitend gekoppelten anderen elektronischen Komponenten (48, 50) angeordnet ist.

10. DEA (18) nach Anspruch 3, ferner umfassend mindestens eine der ersten oder der zweiten Kühlplatte (54), die jeweils aufweisen:
eine Kühlmittelleitung mit einem ersten Ende, das den jeweiligen ersten oder zweiten Einlass (60) bildet, und einem zweiten Ende, das den jeweiligen ersten oder zweiten Auslass (62) bildet; und
geteilte Konstruktion mit jeweiliger oberer und unterer Platte in direktem Kontakt mit der Kühlmittelleitung, die diese dazwischen einfangen.

11. Fluidkühlmittelsystem nach Anspruch 1 oder DEA (18) nach Anspruch 3, wobei die erste Kühlplatte (52, 152) oder die zweite Kühlplatte (54) ferner Plattenteilsegmente mit gemeinsam genutzten ersten und zweiten Einlässen (60) bzw. ersten oder zweiten Auslässen (62) umfasst.

12. Medizinisches Bildgebungssystem, das eine Vielzahl der DEAs (18) nach Anspruch 3 einbezieht, ferner umfassend:
eine Kühleinrichtung (30), die mit und extern zur Gantry (12) gekoppelt ist und eine Kühlmittelzufuhr zum Zuführen von fluidem Kühlmittel zur Gantry (12) und eine Kühlmittelrückführung zum Zurückführen des Kühlmittels zur Kühleinrichtung (30) aufweist;
die Vielzahl von DEAs (18), die mit der Kühleinrichtung (30) gekoppelt sind;
die jeweiligen Gehäuse (40) der DEA (18), die gemeinsam mit einem Träger des Gehäuses (40) einer Gantry (12) des medizinischen Bildgebungssystems gekoppelt sind, wobei die Gehäuse (40) der DEA (18) axial nacheinander um eine Außenseite eines Patiententunnels der Gantry (12), parallel zu einer zentralen Achse, die durch den Patiententunnel definiert wird, ausgerichtet sind;
die jeweiligen Detektorelemente (44) der DEAs (18), die dem Patiententunnel zugewandt und außerhalb von und tangential zu einem Umfang des Patiententunnels ausgerichtet sind, wobei Detektorelementkomponenten radial vom Patiententunnel wegragen;
die erste Kühlplatte (52, 152) in jeder der jeweiligen DEAs (18) in thermisch leitendem, direktem Kontakt mit mindestens einer der Detektorelementkomponenten und im Gehäuse (40) zwischen den Detektorelementkomponenten und den anderen elektronischen Komponenten (48, 50) ausgerichtet;
die zweite Kühlplatte (54) in jeder der jeweiligen DEAs (18), die gegenüberliegende erste und zweite Seiten aufweist, die normal zu dem Umfang des Patiententunnels ausgerichtet sind;
die anderen elektronischen Komponenten (48, 50), die eine Leiterplatte und eine Leistungsversorgung aufweisen, die jeweils thermisch mit der jeweiligen ersten und zweiten Seite der zweiten Kühlplatte (54) gekoppelt sind; und
die jeweiligen ersten Einlässe (56) von allen der ersten Kühlplatten (52, 152) der DEAs (18), die kollektiv Kühlmittel von der Kühlmittelzufuhr aufnehmen und anschließend das Kühlmittel aus ihren jeweiligen ersten Auslässen (58) ablassen, worauf das abgelassene Kühlmittel anschließend kollektiv in den jeweiligen zweiten Einlässen (60) von allen der zweiten Kühlplatten (54) aufgenommen wird und dann aus ihren jeweiligen zweiten Auslässen (62) zu der Kühlmittelrückführung abgelassen wird.

13. Verfahren zum skalierbaren Kühlen einer Gantry (12) einer medizinischen Bildgebungseinrichtung (10), umfassend:
Bereitstellen einer Gantry (12), die einen Patiententunnel bildet;
Bereitstellen einer Kühleinrichtung (30), die mit und extern zur Gantry (12) gekoppelt ist und eine Kühlmittelzufuhr zum Zuführen von fluidem Kühlmittel zur Gantry (12) und eine Kühlmittelrückführung zum Zurückführen des Kühlmittels zur Kühleinrichtung (30) aufweist;
Bereitstellen einer ausgewählten Vielzahl von elektronischen Detektorbaugruppen (DEAs) (18) zur Ausrichtung innerhalb der Gantry (12), wobei jede jeweilige DEA (18) aufweist:
ein Gehäuse (40);
Detektorelemente (44) im Gehäuse (40) zum Detektieren von einfallenden Photonen von elektromagnetischer Strahlung, die von außerhalb des Gehäuses (40) herrührt;
andere elektronische Komponenten (48, 50) im Gehäuse (40), einschließlich einer Leiterplatte und einer Leistungsversorgung;
eine fluidgekühlte erste Kühlplatte (52, 152), die thermisch leitend mit den Detektorelementen (44) gekoppelt ist, zum Kühlen der Detektorelemente (44) und ausgerichtet im Gehäuse (40) zwischen den Detektorelementen (44) und den anderen elektronischen Komponenten (48, 50), wobei die erste Kühlplatte (52, 152) einen ersten Einlass (56) zum Aufnehmen des Kühlmittels von der Kühlmittelzufuhr und einen ersten Auslass (58) zum Ablassen des Kühlmittels zur Kühlmittelrückführung aufweist; und
eine fluidgekühlte zweite Kühlplatte (54) zum Kühlen der anderen elektronischen Komponenten (48, 50), wobei die zweite Kühlplatte (54) gegenüberliegende erste und zweite Seiten aufweist, die jeweils thermisch leitend mit der Leiterplatte auf ihrer ersten Seite und mit der Leistungsversorgung auf ihrer zweiten Seite gekoppelt sind, wobei die zweite Kühlplatte (54) einen zweiten Einlass (60) zum Aufnehmen des Kühlmittels von der Kühlmittelzufuhr und einen zweiten Auslass (62) zum Ablassen des Kühlmittels zu der Kühlmittelrückfuhr aufweist;
die jeweiligen ersten und zweiten Einlässe (60) und Auslässe der ersten und zweiten Kühlplatte (54), die extern außerhalb jedes jeweiligen Gehäuses (40) der DEA (18) zugänglich sind; die Vielzahl von DEAs (18) innerhalb der Gantry (12) ausrichtend, sodass:
ihre jeweiligen Gehäuse (40) der DEA (18) gemeinsam mit einem Träger des Gehäuses (40) der Gantry (12) axial nacheinander um eine Außenseite des Patiententunnels, parallel zu einer zentralen Achse, die durch den Patiententunnel definiert wird, gekoppelt sind; und
ihre jeweiligen Detektorelemente (44) dem Patiententunnel zugewandt sind, tangential zu einem Umfang davon, wobei Komponenten des Detektorelements radial vom Patiententunnel wegragen;
Koppeln des ersten Einlasses (56) der ersten Kühlplatte (52, 152) und des zweiten Einlasses (60) der zweiten Kühlplatte (54) jeder DEA (18), direkt oder indirekt, mit der Kühlmittelzufuhr der Kühleinrichtung (30);
Koppeln des ersten Auslasses (58) der ersten Kühlplatte (52, 152) und des zweiten Auslasses (62) der zweiten Kühlplatte (54) jeder DEA (18), direkt oder indirekt, mit der Kühlmittelrückführung der Kühleinrichtung (30);
Skalieren der Kühlkapazität der externen Kühleinrichtung (30) proportional zu der Anzahl von DEAs (18) in der Gantry (12), die dadurch gekühlt werden; und
Zirkulierenlassen des Kühlmittels zwischen der Gantry (12) und der Kühleinrichtung (30) bei einer Durchflussrate, die eine angegebene stabile Temperaturbandbreite für alle Detektorelemente (44) in jeder der jeweiligen DEAs (18) beibehält.

14. Verfahren zum skalierbaren Kühlen einer Gantry (12) nach Anspruch 13, ferner umfassend:
kollektives Koppeln von allen der ersten Einlässe (56) der ersten Kühlplatten (52, 152) mit der Kühlmittelzufuhr der Kühleinrichtung (30); und
kollektives Koppeln von allen der zweiten Einlässe (60) der zweiten Kühlplatten (54) mit den kollektiven ersten Auslässen (58) der ersten Kühlplatten (52, 152), sodass Kühlmittel von der Kühlmittelzufuhr zunächst durch alle der ersten Kühlplatten (52, 152) fließt, bevor es durch die zweiten Kühlplatten (54) fließt.

15. Verfahren zum skalierbaren Kühlen einer Gantry (12) nach Anspruch 13, wobei die erste Kühlplatte (52, 152) oder die zweite Kühlplatte (54) ferner Plattenteilsegmente mit gemeinsam genutzten ersten und zweiten Einlässen (60) bzw. ersten oder zweiten Auslässen (62) umfasst.

## Revendications

1. Système de refroidissement par fluide pour un appareil d'imagerie médicale (10), comprenant :
un portique (12) formant un tunnel pour patient ;
un appareil de refroidissement (30), couplé au portique (12) et extérieur à celui-ci, ayant une alimentation en réfrigérant pour fournir un fluide réfrigérant au portique (12), et un retour de réfrigérant pour retourner le réfrigérant à l'appareil de refroidissement (30) ;
un ensemble électronique de détection (DEA) (18) à l'intérieur du portique (12), couplé à l'appareil de refroidissement (30), comprenant :
un boîtier (40) ;
des éléments de détection (44) dans le boîtier (40), pour détecter des photons incidents de rayonnements électromagnétiques provenant de l'extérieur du boîtier (40) ;
d'autres composants électroniques (48, 50) dans le boîtier (40) ;
une première plaque de refroidissement refroidie par un fluide (52, 152) couplée de façon thermoconductrice aux éléments de détection (44), pour refroidir les éléments de détection (44), la première plaque de refroidissement (52, 152) ayant une première entrée (56) pour recevoir le réfrigérant depuis l'alimentation en réfrigérant et une première sortie (58) pour évacuer le réfrigérant vers le retour de réfrigérant ; et
une seconde plaque de refroidissement refroidie par un fluide (54) couplée de façon thermoconductrice aux autres composants électroniques (48, 50), pour refroidir les autres composants électroniques (48, 50), la seconde plaque de refroidissement (54) ayant une seconde entrée (60) pour recevoir le réfrigérant depuis l'alimentation en réfrigérant et une seconde sortie (62) pour évacuer le réfrigérant vers le retour de réfrigérant ;
le système de refroidissement par fluide comprenant en outre :
les éléments de détection (44) ayant des composants d'éléments de détection qui font saillie à partir d'eux, et qui définissent un premier profil de surface ;
la première plaque de refroidissement (52, 152) en contact direct thermoconducteur avec au moins une partie du premier profil de surface des composants d'éléments de détection ;
au moins l'un des autres composants électroniques (48, 50) définissant un second profil de surface ; et
la seconde plaque de refroidissement (54) en contact direct thermoconducteur avec au moins une partie du second profil de surface de l'au moins un des autres composants électroniques (48, 50).

2. Système de refroidissement par fluide selon la revendication 1, comprenant en outre :
les éléments de détection (44) présents dans le DEA (18) faisant face au tunnel pour patient, les éléments de détection (44) ayant des composants d'éléments de détection faisant saillie radialement à partir du tunnel pour patient ;
la première plaque de refroidissement (52, 152) en contact direct thermoconducteur avec au moins l'un des composants d'éléments de détection et orientée dans le boîtier (40) entre les composants d'éléments de détection et les autres composants électroniques (48, 50) ; et
la seconde plaque de refroidissement (54) en contact direct thermoconducteur avec les autres composants électroniques (48, 50).

3. Ensemble électronique de détection (DEA) (18) pour appareil d'imagerie médicale (10), comprenant :
un boîtier (40) ;
des éléments de détection (44) contenus dans le boîtier (40), pour détecter des photons incidents de rayonnements électromagnétiques provenant de l'extérieur du boîtier (40) ;
d'autres composants électroniques (48, 50) dans le boîtier (40) ;
une première plaque de refroidissement refroidie par un fluide (52, 152) couplée de façon thermoconductrice aux éléments de détection (44), pour refroidir les éléments de détection (44), la première plaque de refroidissement (52, 152) ayant une première entrée (56) pour recevoir le fluide réfrigérant et une première sortie (58) pour évacuer le réfrigérant ;
une seconde plaque de refroidissement refroidie par un fluide (54) couplée de façon thermoconductrice aux autres composants électroniques (48, 50), pour refroidir les autres composants électroniques (48, 50), la seconde plaque de refroidissement (54) ayant une seconde entrée (60) pour recevoir le réfrigérant et une seconde sortie (62) pour évacuer le réfrigérant ;
les premières et secondes entrées (60) et sorties respectives accessibles à l'extérieur du boîtier (40) ;
le DEA (18) comprenant en outre :
les éléments de détection (44) ayant des composants d'éléments de détection qui font saillie à partir d'eux, et qui définissent un premier profil de surface ;
la première plaque de refroidissement (52, 152) en contact direct thermoconducteur avec au moins une partie du premier profil de surface des composants d'éléments de détection ;
au moins l'un des autres composants électroniques (48, 50) définissant un second profil de surface ; et
la seconde plaque de refroidissement (54) en contact direct thermoconducteur avec au moins une partie du second profil de surface de l'au moins un des autres composants électroniques (48, 50).

4. Système de refroidissement par fluide selon la revendication 1 ou DEA (18) selon la revendication 3, comprenant en outre
la première plaque de refroidissement (52, 152) définissant un premier profil de surface qui est une image miroir du premier profil de surface des éléments de détection (44), les premiers profils de surface respectifs étant en contact direct thermoconducteur entre eux ; et
la seconde plaque de refroidissement (54) définissant un second profil de surface qui est une image miroir du second profil de surface de l'au moins un des autres composants électroniques (48, 50), les seconds profils de surface respectifs étant en contact direct thermoconducteur entre eux.

5. Système de refroidissement par fluide selon la revendication 2 ou DEA (18) selon la revendication 4, comprenant en outre la première sortie (58) de la première plaque de refroidissement (52, 152) en communication fluide avec la seconde entrée (60) de la seconde plaque de refroidissement (54), de telle sorte que le réfrigérant s'écoule tout d'abord à travers la première plaque de refroidissement (52, 152), puis de manière séquentielle, en cascade, à travers la seconde plaque de refroidissement (54).

6. DEA (18) selon la revendication 3, comprenant en outre :
les éléments de détection (44) présents dans le DEA (18), exposés à l'extérieur du boîtier (40), les composants d'éléments de détection faisant saillie vers l'intérieur du boîtier (40) ;
la première plaque de refroidissement (52, 152) en contact direct thermoconducteur avec au moins l'un des composants d'éléments de détection et orientée dans le boîtier (40) entre les composants d'éléments de détection et les autres composants électroniques (48, 50) ; et
la seconde plaque de refroidissement (54) en contact direct thermoconducteur avec les autres composants électroniques (48, 50).

7. Système de refroidissement par fluide selon la revendication 5 ou DEA (18) selon la revendication 6, comprenant en outre
la seconde plaque de refroidissement (54) ayant des première et seconde faces opposées ; et
les autres composants électroniques (48, 50) incluant une carte de circuits imprimés et une alimentation électrique, couplées thermiquement respectivement aux première et seconde faces respectives de la seconde plaque de refroidissement (54).

8. DEA (18) selon la revendication 3, comprenant en outre :
la seconde plaque de refroidissement (54) ayant des première et seconde faces opposées ; et
les autres composants électroniques (48, 50) incluant une carte de circuits imprimés et une alimentation électrique, couplées thermiquement respectivement aux première et seconde faces respectives de la seconde plaque de refroidissement (54).

9. DEA (18) selon la revendication 3, comprenant en outre un matériau thermoconducteur interposé entre la première plaque de refroidissement (52, 152) et ses éléments de détection couplés de façon thermoconductrice (44) et/ou entre la seconde plaque de refroidissement (54) et ses autres composants électroniques (48, 50) couplés de façon thermoconductrice.

10. DEA (18) selon la revendication 3, comprenant en outre au moins l'une des première ou seconde plaques de refroidissement (54) ayant respectivement :
un tuyau de réfrigérant ayant une première extrémité formant la première ou seconde entrée respective (60) et une seconde extrémité formant la première ou la seconde sortie respective (62) ; et
des plaques de dessus et de dessous respectives à construction fendue en contact direct avec le tuyau de réfrigérant, en le capturant entre elles.

11. Système de refroidissement par fluide selon la revendication 1 ou DEA (18) selon la revendication 3, la première plaque de refroidissement (52, 152) ou la seconde plaque de refroidissement (54) comprenant en outre des sous-segments de plaque ayant des premières ou secondes entrées (60) et des premières ou secondes sorties (62) respectives partagées en commun.

12. Système d'imagerie médicale incorporant une pluralité de DEA (18) selon la revendication 3, comprenant en outre :
un appareil de refroidissement (30), couplé au portique (12) et extérieur à celui-ci, ayant une alimentation en réfrigérant pour fournir un fluide réfrigérant au portique (12), et un retour de réfrigérant pour retourner le réfrigérant à l'appareil de refroidissement (30) ;
la pluralité de DEA (18) couplée à l'appareil de refroidissement (30) ;
les boîtiers (40) des DEA respectifs (18) étant couplés en commun à un support de boîtier (40) d'un portique (12) du système d'imagerie médicale, les boîtiers (40) des DEA (18) étant orientés axialement en séquence autour d'un extérieur d'un tunnel pour patient du portique (12), parallèle à un axe central défini par le tunnel pour patient ;
les éléments de détection respectifs (44) présents dans les DEA (18) faisant face au tunnel pour patient, orientés vers l'extérieur d'une circonférence du tunnel pour patient et tangentiels à celle-ci, avec des composants d'éléments de détection faisant saillie radialement à partir du tunnel pour patient ;
la première plaque de refroidissement (52, 152) de chacun des DEA respectifs (18), en contact direct thermoconducteur avec au moins l'un des composants d'éléments de détection et orientée dans le boîtier (40) entre les composants d'éléments de détection et les autres composants électroniques (48, 50) ;
la seconde plaque de refroidissement (54) de chacun des DEA respectifs (18) ayant des première et seconde faces opposées, orientée perpendiculairement à la circonférence du tunnel pour patient ;
les autres composants électroniques (48, 50) incluant une carte de circuits imprimés et une alimentation électrique, couplées thermiquement respectivement aux première et seconde faces respectives de la seconde plaque de refroidissement (54) ; et
les premières entrées respectives (56) de toutes les premières plaques de refroidissement (52, 152) des DEA (18)_recevant collectivement du réfrigérant depuis l'alimentation en réfrigérant puis déchargeant le réfrigérant par leurs premières sorties respectives (58), le réfrigérant déchargé étant ensuite reçu collectivement dans les secondes entrées respectives (60) de la totalité de secondes plaques de refroidissement (54) puis déchargé par leurs secondes sorties respectives (62) vers le retour de réfrigérant.

13. Procédé de refroidissement ajustable d'un portique (12) d'un appareil d'imagerie médicale (10), comprenant :
la fourniture d'un portique (12) formant un tunnel pour patient ;
la fourniture d'un appareil de refroidissement (30), couplé au portique (12) et extérieur à celui-ci, ayant une alimentation en réfrigérant pour fournir un fluide réfrigérant au portique (12), et un retour de réfrigérant pour retourner le réfrigérant à l'appareil de refroidissement (30) ;
la fourniture d'une pluralité sélectionnée d'ensembles électroniques de détection (DEA) (18) pour l'orientation dans le portique (12), chaque DEA respectif (18) ayant :
un boîtier (40) ;
des éléments de détection (44) dans le boîtier (40), pour détecter des photons incidents de rayonnements électromagnétiques provenant de l'extérieur du boîtier (40) ;
d'autres composants électroniques (48, 50) dans le boîtier (40), incluant une carte de circuits imprimés et une alimentation électrique ;
une première plaque de refroidissement refroidie par un fluide (52, 152) couplée de façon thermoconductrice aux éléments de détection (44), pour refroidir les éléments de détection (44) et orientée dans le boîtier (40) entre les éléments de détection (44) et les autres composants électroniques (48, 50), la première plaque de refroidissement (52, 152) ayant une première entrée (56) pour recevoir le réfrigérant depuis l'alimentation en réfrigérant et une première sortie (58) pour évacuer le réfrigérant vers le retour de réfrigérant ; et
une seconde plaque de refroidissement refroidie par un fluide (54) pour refroidir les autres composants électroniques (48, 50), la seconde plaque de refroidissement (54) ayant des première et seconde faces opposées, respectivement couplée de façon thermoconductrice à la carte de circuits imprimés sur sa première face et à l'alimentation électrique sur sa seconde face, la seconde plaque de refroidissement (54) ayant une seconde entrée (60) pour recevoir le réfrigérant depuis l'entrée de réfrigérant et une seconde sortie (62) pour décharger le réfrigérant vers le retour de réfrigérant ;
les première et seconde entrées (60) et sorties respectives des première et seconde plaques de refroidissement (54) étant accessibles depuis extérieur en dehors de chaque boîtier (40) de DEA (18) respectif ; orientant la pluralité de DEA (18) dans le portique (12), de telle sorte que :
leurs boîtiers (40) de DEA respectifs (18) soient couplés en commun à un support de boîtier (40) du portique (12), axialement en séquence autour d'un extérieur du tunnel pour patient, parallèlement à un axe central défini par le tunnel pour patient ; et
leurs éléments de détection respectifs (44) soient disposés face au tunnel pour patient, tangentiellement à une circonférence de celui-ci, avec des composants de l'élément de détection faisant saillie radialement à partir du tunnel pour patient ;
le couplage de la première entrée (56) de la première plaque de refroidissement (52, 152) et de la seconde entrée (60) de la seconde plaque de refroidissement (54) de chaque DEA (18) directement ou indirectement à l'alimentation en réfrigérant de l'appareil de refroidissement (30) ;
le couplage de la première sortie (58) de la première plaque de refroidissement (52, 152) et de la seconde sortie (62) de la seconde plaque de refroidissement (54) de chaque DEA (18) directement ou indirectement au retour de réfrigérant de l'appareil de refroidissement (30) ;
l'ajustement de la capacité de refroidissement de l'appareil de refroidissement externe (30) en proportion du nombre de DEA (18) dans le portique (12) qui sont refroidis ensemble ; et
la circulation du réfrigérant entre le portique (12) et l'appareil de refroidissement (30) à un débit qui maintient une plage de températures stable spécifiée pour tous les éléments de détection (44) dans chacun des DEA respectifs (18).

14. Procédé de refroidissement ajustable d'un portique (12) selon la revendication 13, comprenant en outre :
le couplage collectif de la totalité des premières entrées (56) des premières plaques de refroidissement (52, 152) à l'alimentation en réfrigérant de l'appareil de refroidissement (30) ; et
le couplage collectif de la totalité des secondes entrées (60) des secondes plaques de refroidissement (54) aux premières sorties collectives (58) des premières plaques de refroidissement (52, 152), de telle sorte que le réfrigérant provenant de l'alimentation en réfrigérant s'écoule tout d'abord à travers la totalité des premières plaques de refroidissement (52, 152) avant de s'écouler à travers les secondes plaques de refroidissement (54).

15. Procédé de refroidissement ajustable d'une portique (12) selon la revendication 13, la première plaque de refroidissement (52, 152) ou la seconde plaque de refroidissement (54) comprenant en outre des sous-segments de plaque ayant des premières ou secondes entrées (60) et des premières ou secondes sorties (62) respectives partagées en commun.
